# EUROPEAN PATENT APPLICATION

(11) **EP 2 055 379 A1**
(43) Date of publication of application: **06.05.2009**
(21) Application number: 08154750.7
(22) Date of filing: 17.04.2008
(51) Int. Cl.: B01J 23/745, B01J 23/755, B01J 25/02, C07C 213/00

(54) **Raney-nickel-iron catalyst, its preparation and a method to produce L-norephedrine by hydrogenating L-phenylacetylcarbinol-oxime with said catalyst**

(30) Priority: 29.10.2007 IN MU21442007
(71) Applicant: Emmellen Biotech Pharmaceuticals Ltd., 400076 Mumbai (IN)
(72) Inventor: Joshi, Sunil Vaman, 402309 RAIGAD (IN)
(74) Representative: Le Coupanec, Pascale A.M.P.

(57) **Abstract**

The present invention provides a novel hydrogenation catalyst, process of preparing the catalyst and process for the preparation of optically active L-norephedrine, [(1R,2S)-2-amino-1-phenyl-1-propanol] by a catalytic hydrogenation process, said catalyst comprising of finely divided nickel metal containing a metal from group III A of the periodic table as an activator and a metal from group VI B or VIII as promoter.

## Description

### Field of invention:

The present invention relates to a novel catalyst for the hydrogenation process, process for preparation of the said catalyst and an efficient process for preparation of L-norephedrine, [(1R,2S)-2-amino-1-phenyl-1-propanol] by a catalytic hydrogenation process.

More particularly the present invention relates to a novel hydrogenation catalyst comprising of finely divided nickel metal containing a metal from group III A of the periodic table as an activator and a metal from group VI B or VIII as promoter, for the hydrogenation process.

The present invention further relates to a process for preparation of the said hydrogenation catalyst.

More particularly the present invention relates to an efficient process for preparation of optically active L-norephedrine [(1R,2S)-2-amino-1-phenyl-1-propanol] which is substantially free from its optical antipodes and diastereomeric impurities by a catalytic hydrogenation process employing the said novel catalyst.

### Background of the invention:

L-Norephedrine is an adrenergic drug used in many preparations to relieve allergic reactions or respiratory infections. L-Norephedrine [(1R,2S)-2-amino-1-phenyl-1-propanol] is prepared by the reduction/hydrogenation of L-Phenylacetylcarbinol-oxime (L-PAC).

Well known highly active hydrogenation catalysts are platinum group metals, particularly platinum, palladium, rhodium and ruthenium. Highly active catalysts operate at lower temperatures and lower pressures of H₂. Non-precious metal catalysts, especially those based on nickel (such as Raney nickel and Urushibara nickel) have also been developed as economical alternatives.

WO 2005/100299 describes a novel and efficient process for preparation of L-erythro-2-amino-1-phenyl-1-propanol substantially free from optical antipodes and diastereomeric impurities. The process involves conversion of L-phenylacetylcarbinol to its oxime derivative by treatment with hydroxylamine salts and reducing the so formed oxime with a catalyst comprising nickel and aluminium metals. (Scheme I)

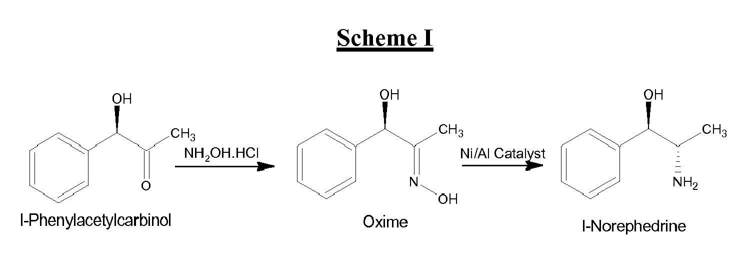

WO 2005/100299 provides a comprehensive summary of the literature on the subject matter. The only references that mention reduction of L-Phenylacetylcarbinol-oxime are given below.

D.H.Hey, J.Chem. Soc. 1232, (1930) describes preparation of d1-PAC oxime by reaction of dl-mandelamide with methyl magnesium bromide and reduction of dl-PAC oxime with sodium amalgam.

W.H.Hartung and J.C.Munch, J.Am.Chem.Soc., 51, 2262-2266 (1929), describes formation of dl-PAC oxime and reduction with sodium amalgam.

GB 365,535 discloses a process for preparation of L-PAC oxime and its reduction by hydrogenation over 'precious metal' catalysts.

DE 587,586 discloses preparation of L-PAC oxime and its reduction by hydrogenation over 'precious metal' catalyst.

DE 1,014,553 discloses preparation of L-PAC oxime and its reduction with amalgamated aluminium.

JP 05-004948 discloses reduction of L-PAC oxime using Rh complexes of chiral substituted ferrocenyl phosphine ligands as hydrogenation catalysts.

Thus there is needed for catalytic hydrogenation of L-Phenylacetylcarbinol-oxime to give optically active L-norephedrine [(1R, 2S)-2-amino-1-phenyl-1-propanol] in a manner that it does not generate effluents- either liquid or gaseous, which may be harmful and the used catalyst could be filtered and recycled.

### Objectives of the invention:

It is thus an object of the invention to provide a novel hydrogenation catalyst.

It is a further object of the present invention to provide a novel catalyst for catalytic hydrogenation of L-Phenylacetylcarbinol-oxime to give optically active L-norephedrine [(1R,2S)-2-amino-1-phenyl-1-propanol].

Yet another object of this invention is to provide a process for the preparation of the said novel catalyst.

Yet another object of the present invention is to provide a process for reduction of L-Phenylacetylcarbinol-oxime to give optically active L-norephedrine [(1R,2S)-2-amino-1-phenyl-1-propanol] by a catalytic hydrogenation process.

It is a further object of the present invention to provide a process for preparation of optically active L-norephedrine [(1R,2S)-2-amino-1-phenyl-1-propanol], which is substantially free from its optical antipodes and diastereomeric impurities.

Yet another object of this invention is to provide a process of catalytic hydrogenation of L-Phenylacetylcarbinol-oxime to give optically active L-norephedrine [(1R,2S)-2-amino-1-phenyl-1-propanol] that does not generate effluents- either liquid or gaseous.

Yet another object of this invention is to provide a process of catalytic hydrogenation of L-Phenylacetylcarbinol-oxime to give optically active L-norephedrine [(1R,2S)-2-amino-1-phenyl-1-propanol] that is safe to operate.

### Summary of the invention:

The present invention provides a novel hydrogenation catalyst comprising finely divided nickel metal containing a metal from group III A of the periodic table as an activator and a metal from group VI B or VIII as promoter,

The present invention aims at the preparation of catalyst comprising of and catalytic hydrogenation of L-Phenylacetylcarbinol-oxime to produce L-norephedrine.

### Detailed description of the invention:

According to the present invention, there is provided novel compositions of catalyst for catalytic hydrogenation of L-Phenylacetylcarbinol-oxime to give optically active L-norephedrine [(1R,2S)-2-amino-1-phenyl-1-propanol], said catalyst comprising of finely divided nickel metal containing a metal from group III A of the periodic table as an activator and a metal from group VI B or VIII as promoter.

In a preferred embodiment of the invention, the L-Phenylacetylcarbinol-oxime (which may be prepared e.g. as per the process disclosed in WO 2005/100299) is dissolved in a solvent comprising either water alone or a mixture of water and a C-1 to C-3 lower alcohol and having an alkaline pH, wherein the said C-1 to C-3 lower alcohol is contained in the solvent mixture to the extent between 10% (v/v) to 100% (v/v), preferably between 50% (v/v) to 90% (v/v).

This solution of L-Phenylacetylcarbinol-oxime is hydrogenated in a high-pressure autoclave in the presence of a novel hydrogenation catalyst.

The strength of L-Phenylacetylcarbinol-oxime in the hydrogenation mass may be in the range of 1% to 25% (w/v), preferably between 10% to 16 % (w/v).

The pH of the solution is rendered alkaline by addition of alkali metal hydroxides, preferably NaOH.

The hydrogenation is carried out at hydrogen pressure and maintained between 1 kg/cm² to 15 kg/cm², preferably between 3 kg/cm² and 10 kg/cm².

The temperature of hydrogenation reaction is maintained between ambient to 100°C.

The quantity of catalyst used is between 0.5 % and 5 % (w/v) of the reaction mixture. After the absorption of hydrogen has ceased, as indicated by the rate of drop in pressure, the reaction mass is filtered to remove the catalyst.

The filtered catalyst retains its activity and can be recycled into the next batch of hydrogenation with addition of an appropriate top-up quantity of fresh catalyst to account for the handling loss of the catalyst.

The filtrate is optionally concentrated under reduced pressure to recover the organic solvents, if used, and processed further to recover the product, crude L-norephedrine.

This crude product consists chiefly of the 1-erythro isomer of L-norephedrine together with some of the diastereomer impurity, the 1-threo isomer, L-norpseudoephedrine. Thus this process of hydrogenation with the said novel hydrogenation catalyst does not produce liquid or gaseous effluents; the catalyst being recyclable, there are no waste materials after the hydrogenation.

The catalyst used for the reaction is a novel combination of metals and comprises finely divided nickel metal containing a metal from group III A of the periodic table as an activator and a metal from group VI B or VIII as promoter.

The metal from group III A of the periodic table may be aluminium. The metal from group VIII of the periodic table may be iron or cobalt, and the metal from group VI B of the periodic table may be molybdenum or chromium.

The activator may be present to the extent of 2 to 15 % by weight and the promoter may be present to the extent of 1 to 5 % by weight of the catalyst. The said catalyst is prepared from an initial mixture of metals in alloy form by a process that leaches out substantial portion of the activator metal but does not remove the promoter metal from the initial mixture.

### Processing of the catalyst:

The process of converting the initial mixture of metals into the abovementioned catalyst comprises
(i) contacting the said initial metal mixture in a finely pulverized state with a solution of alkali metal hydroxide and maintaining the said contact till a desired level of metal leaching and a desired level of catalytic activity is achieved.
(ii) containing alkali metal hydroxide in the solution as 10% to 40% by weight.
(iii) comprising nickel metal of the initial metal mixture to the extent between 30 % and 60% by weight.
(iv) adding the metal as activator present in the initial metal mixture from 40% to 70% by weight.
(v) adding the metal as promoter present in the initial metal mixture from 1% to 5% by weight.
(vi) keeping the volume of the solution of alkali metal hydroxide between 2 to 20 times the weight of initial metal mixture
(vii) keeping the temperature of the contacting process between 50°C and 110°C.
(viii) continuing the contacting process at the contacting temperature and monitoring the activity of the catalyst produced.
(ix) continuing the contact process until the content of the activator added to the initial metal mixture in the range between 2 to 15 % by weight and an activity level of hydrogen absorption of at least 80 ml/min per gram of catalyst is achieved.
(x) measuring the catalyst activity by measuring the rate of hydrogen absorption when hydrogenation of nitrobenzene is carried out under standard test conditions.
(xi) washing the catalyst slurry with water by decantation till it is free from alkali.
(xii) producing the material which is essentially the conventional Raney nickel by abovementioned process when a metal mixture is a 1:1 mixture of nickel and aluminium metals in an alloy form.

However, when Raney nickel is used as catalyst for the abovementioned process of hydrogenation of L-Phenylacetylcarbinol-oxime, the diastereomeric and enantiomeric purity of L-norephedrine obtained is adversely affected. Besides, the conversion of L-Phenylacetylcarbinol-oxime to norephedrine bases is also very poor. However, the inventors found, quite surprisingly, the new catalyst prepared as detailed above and used as catalyst for hydrogenation of L-Phenylacetylcarbinol-oxime gave excellent conversion of L-Phenylacetylcarbinol-oxime to L-norephedrine and excellent diastereomeric and enantiomeric purity of the L-norephedrine produced.

Pure L-norephedrine is obtained from the crude product by treatment with an organic acid in aqueous media, or a lower aliphatic alcohol, or a mixture of water and one of these alcohols, or a mixture of two of these alcohols, at temperature between 0°C and the boiling point of the solvent at atmospheric pressure. The desired isomer, L-norephedrine, which forms less soluble salt, is filtered off. The salt containing desired isomer is decomposed by treatment with a base and the base form of the desired isomer is extracted using an organic solvent. Pure L-norephedrine is then isolated by evaporation of the solvent under reduced pressure.

The pure L-norephedrine thus isolated in the base form may be converted to salts with organic or inorganic acids by treatment with appropriate organic or inorganic acids.

The organic acids used for separation of the erythro and threo isomers is preferably selected from a group comprising of acetic acid, propionic acid, butyric acid, isobutyric acid, oxalic acid, malonic acid, succinic acid, cyclohexanecarboxylic acid, maleic acid, benzoic acid, p-toluic acid, methanesulphonic acid, benzenesulphonic acid or p-toluenesulphonic acid.

The solvent used for this separation reaction is either water or a lower aliphatic alcohol preferably selected from a group comprising methanol, ethanol, isopropyl alcohol, n-butanol, 2-butanol and tert-butanol or a mixture of water and one of these alcohols or a mixture of two of these alcohols.

The base used for rendering alkaline the reaction mixture and for decomposition of the organic acid salt of L-norephedrine is preferably selected from a group comprising sodium carbonate, sodium hydrogen carbonate, sodium hydroxide, sodium acetate, potassium carbonate, potassium hydroxide, potassium acetate, barium hydroxide, preferably sodium hydroxide, potassium hydroxide or sodium hydrogen carbonate.

The base is present in the reaction mass to the extent of between 5% (w/v) to 25% (w/v).

The solvent used for extraction of pure L-norephedrine base is selected from a group comprising toluene, benzene, 1,2-dichloroethane, di-n-butyl ether, diethyl ether, methyl tert-butyl ether, monochlorobenzene, n-butanol, chloroform and dichloromethane.

The details of the invention, its objects and advantages are explained hereunder in greater detail in relation to non-limiting exemplary illustrations. The examples are merely illustrative and do not limit the teachings of this invention and it would be obvious that various modifications or changes in the procedural steps by those skilled in the art without departing from the scope of the invention and shall be consequently encompassed within the ambit and spirit of this approach and scope thereof.

### Example 1:

### Preparation of novel hydrogenation catalyst:

A solution of 20 g of sodium hydroxide in 70 ml water is heated to 50-60°C. 5 g of metal mixture containing 30 % nickel, 65 % aluminium and 5 % iron was added to it in small portions while stirring vigorously. After the addition is over, the mixture is heated to boiling for 30 minutes. A small aliquot portion was washed by decantation and its hydrogenation activity was checked on standard hydrogenation apparatus by hydrogenation of nitrobenzene. The activity was 85 ml /min per gram of catalyst. The slurry was cooled and washed with water by decantation till neutral.

### Example 2:

### Hydrogenation of L-Phenylacetylcarbinol-oxime with novel hydrogenation catalyst:

11 kg of L-Phenylacetylcarbinol-oxime as a solution in 200 L of water-methanol mixture (30:70) was mixed with 6 kg of sodium hydroxide. The solution was charged to a high-pressure autoclave equipped with heating jacket and agitator. 5.5 kg of the novel hydrogenation catalyst (prepared as e.g. example 1) as a slurry in water was charged to the autoclave. The autoclave lid was securely closed. The autoclave was evacuated and vacuum released with nitrogen. This evacuation and releasing of vacuum was repeated three times. Then the same sequence of evacuation and releasing of vacuum with hydrogen was repeated two times. Finally, the autoclave was pressurized with hydrogen upto 7 kg/cm² pressure. The temperature was maintained between ambient and 65°C by application of steam/cooling water to the autoclave jacket as necessary. The pressure was maintained between 6.0 kg/cm² and 7.0 kg/cm² until a drop in pressure was no longer observed. The agitation was stopped, reaction mass allowed to settle and decanted from the catalyst by passage through a filter. The catalyst was washed with methanol by decantation and the washings were filtered. The combined filtrate and washings were charged into a 500-L SS kettle and methanol was distilled from it under reduced pressure upto 95-100°C liquid temperature. The concentrate was cooled and extracted with toluene. The toluene extract was evaporated under vacuum.

This gave 10 kg of L-norephedrine base. This was dissolved in 100 L of absolute ether and treated with ethanolic hydrogen chloride to give L-norephedrine HCl.
m.p. 171-172°C, [ ]_{D}²⁵ : -32.5° (5 % in water)
Diastereomeric purity (HPLC): L-norephedrine: 97.5 %, L-norpseudoephedrine: 2.5%

### Comparative Example 2:

Hydrogenation of L-Phenylacetylcarbinol-oxime with commercial Raney nickel catalyst: 11 kg of L-Phenylacetylcarbinol-oxime as a solution in 200 L of water-methanol mixture (30:70) was mixed with 6 kg of sodium hydroxide. The solution was charged to a high-pressure autoclave equipped with heating jacket and agitator. 5.5 kg of the commercial Raney nickel catalyst as slurry in water was charged to the autoclave. The autoclave lid was securely closed. The autoclave was evacuated and vacuum released with nitrogen. This evacuation and releasing of vacuum was repeated three times. Then the same sequence of evacuation and releasing of vacuum with hydrogen was repeated two times. Finally, the autoclave was pressurized with hydrogen upto 7 kg/cm² pressure. The temperature was maintained between ambient and 65°C by application of steam/cooling water to the autoclave jacket as necessary. The pressure was maintained between 6.0 kg/cm² and 7.0 kg/cm² until a drop in pressure was no longer observed. The agitation was stopped, reaction mass allowed to settle and decanted from the catalyst by passage through a filter. The catalyst was washed with methanol by decantation and the washings were filtered. The combined filtrate and washings were charged into a 500-L SS kettle and methanol was distilled from it under reduced pressure upto 95-100°C liquid temperature. The concentrate was cooled and extracted with toluene. The toluene extract was evaporated under vacuum.

This gave 10 kg of crude L-norephedrine base. This was dissolved in 100 L of absolute ether and treated with ethanolic hydrogen chloride. The reaction mass did not give crystalline L-norephedrine HCI. HPLC analysis of the crude L-norephedrine base revealed that it contained L-norephedrine and L-norpseudoephedrine bases in 55:45 ratio, in addition to several other peaks. Besides, a non-aqueous titration with 0.1 N perchloric acid showed only about 65 % of basic component of mol. wt. 151.20 (mol. wt of L-norephedrine / L-norpseudoephedrine).

## Claims

1. A hydrogenation catalyst comprising finely divided nickel, a metal from group IIIA of the periodic table as an activator and a metal from group VIB or VIII as promoter.

2. The catalyst as claimed in claim 1 wherein the metal from group III A of the periodic table is in an amount of 2 % to 15 % by weight of the catalyst.

3. The catalyst as claimed in claim 1 or 2 wherein the metal from group VI B or VIII of the periodic table is in an amount of 1 % to 5 % by weight of the catalyst.

4. The catalyst according to anyone of the previous claims wherein the metal from group III A of the periodic table is aluminium.

5. The catalyst according to anyone of the previous claims wherein the metal from group VIII of the periodic table is chosen from iron or cobalt.

6. The catalyst according to anyone of the previous claims wherein the metal from group VI B of the periodic table is chosen from molybdenum or chromium.

7. A process for the preparation of novel hydrogenation catalyst as claimed in any of claims 1 to 6 comprising the steps:
(i) mixing of an initial metal mixture comprising nickel, a metal from group III A of the periodic table added as activator and a metal from group VI B or VIII of the periodic table added as promoter ; and
(ii) a solution of alkali metal hydroxide until the content of the activator added to the initial metal mixture in the range between 2 to 15 % by weight and an activity level of hydrogen absorption of at least 80 ml/min per gram of catalyst is achieved.

8. The process as claimed in claim 7 wherein the initial metal mixture contains nickel metal between 30 % and 60 % by weight.

9. The process according to anyone of the claims 7 or 8, wherein the initial metal mixture contains 30% nickel, 65% aluminium and 5% iron.

10. A process for preparation of L-norephedrine from L-Phenylacetylcarbinol-oxime comprising hydrogenating L-Phenylacetylcarbinol-oxime in an alkaline solution in the presence of a hydrogenation catalyst as claimed in claims 1 to 6.

11. The process as claimed in claim 10, wherein the solvent used for dissolving L-Phenylacetylcarbinol-oxime is either water alone or a mixture of water and a C-1 to C-3 lower alcohol, wherein the said C-1 to C-3 lower alcohol is contained in the solvent mixture to the extent between 10 % (v/v) to 100 % (v/v), preferably between 50 % (v/v) to 90 % (v/v).

12. The process as claimed in claim 11, wherein the solvent used for the process is either water or a lower aliphatic alcohol selected from a group comprising methanol, ethanol, isopropyl alcohol, n-butanol, 2-butanol and tert-butanol or a mixture of water and one of these alcohols and a mixture of two of these alcohols.

13. The process according to anyone of the claims 10 to 12, wherein the strength of Phenylacetylcarbinol-oxime in the hydrogenation mass is between 1% to 25% (w/v), preferably between 10% to 16% (w/v).

14. The process according to anyone of the claims 10 to 13,wherein the reaction mixture is rendered alkaline by use of bases selected from a group comprising sodium carbonate, sodium hydrogen carbonate, sodium hydroxide, sodium acetate, potassium carbonate, potassium hydroxide, potassium acetate, barium hydroxide, preferably sodium hydroxide or potassium hydroxide.

15. The process according to anyone of the claims 10 to 14, wherein the reaction mixture is rendered alkaline by use of bases present in the reaction mass to the extent of between 5% (w/v) to 25% (w/v).

16. The process according to anyone of the claims 10 to 15, wherein the quantity of the said hydrogenation catalyst used is between 0.5% to 5% (w/v) of the reaction mixture.

17. The process according to anyone of the claims 10 to 16, wherein the hydrogen pressure is maintained between 1 kg/cm² and 15 kg/cm², preferably between 3 kg/cm² and 10 kg/cm².

18. The process according to anyone of the claims 10 to 17, wherein the temperature of hydrogenation reaction is maintained between ambient to 100°C.

19. The process according to anyone of the claims 10 to 18, wherein the solvent is water-methanol mixture, the base is sodium hydroxide, the temperature of hydrogenation reaction is maintained between ambient and 65°C ant the hydrogen pressure is maintained between 6.0 kg/cm² and 7.0 kg/cm².

20. The process according to anyone of the claims 10 to 19, wherein the hydrogenation catalyst used in the process is a recovered catalyst from a previous batch of hydrogenation with addition of an appropriate top-up quantity of fresh catalyst to account for handling loss.

21. The process according to anyone of the claims 10 to 20, wherein the crude L-norephedrine is treated with an organic acid in the presence of a solvent and the salt formed is decomposed with a base to get pure L-norephedrine.

22. The process as claimed in claim 21 wherein the organic acid is selected from a group comprising acetic acid, propionic acid, butyric acid, isobutyric acid, oxalic acid, malonic acid, succinic acid, cyclohexanecarboxylic acid, maleic acid, benzoic acid, p-toluic acid, methanesulphonic acid, benzenesulphonic acid and p-toluenesulphonic acid.

23. The process according to anyone of the claims 21 to 22, wherein the temperature of reaction of crude L-norephedrine with organic acid is between 0°C and the boiling point of the solvent at atmospheric pressure.

24. The process according to anyone of the claims 21 to 23, wherein the base used for decomposition of the salt of pure L-norephedrine with organic acid is selected from a group comprising sodium carbonate, sodium hydrogen carbonate, sodium hydroxide, sodium acetate, potassium carbonate, potassium hydroxide, potassium acetate, barium hydroxide, preferably sodium hydroxide, potassium hydroxide or sodium hydrogen carbonate.
